(19) Europäisches Patentamt
European Patent Office
Office européen des brevets

(11) **EP 3 881 753 B1**

(12) **EUROPÄISCHE PATENTSCHRIFT**

(45) Veröffentlichungstag und Bekanntmachung des Hinweises auf die Patenterteilung:
**04.06.2025   Patentblatt 2025/23**

(21) Anmeldenummer: **19885864.9**

(22) Anmeldetag: **15.11.2019**

(51) Internationale Patentklassifikation (IPC):
**A61B 5/00** (2006.01)

(52) Gemeinsame Patentklassifikation (CPC):
**A61B 5/7257; A61B 5/0531;** A61B 2562/0223

(86) Internationale Anmeldenummer:
**PCT/RU2019/000822**

(87) Internationale Veröffentlichungsnummer:
**WO 2020/101537 (22.05.2020 Gazette 2020/21)**

(54) **VORRICHTUNG ZUR ERZEUGUNG EINES ELEKTROMAGNETISCHEN FELDES UND MESSUNG DER ABSORPTION DAVON DURCH EIN LEITFÄHIGES MEDIUM**

DEVICE FOR GENERATING AN ELECTROMAGNETIC FIELD AND MEASURING THE ABSORPTION THEREOF BY A CONDUCTIVE MEDIUM

INSTRUMENT POUR GÉNÉRER UN CHAMP ÉLECTROMAGNÉTIQUE ET MESURER SON ABSORPTION PAR UN MILIEU CONDUCTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.11.2018   RU 2018140550**

(43) Veröffentlichungstag der Anmeldung:
**22.09.2021   Patentblatt 2021/38**

(73) Patentinhaber: **OBSCHESTVO S OGRANICHENNOY OTVETSTVENNOSTYU "CYBERDOC"**
Moscow, 115419 (RU)

(72) Erfinder:
• **KOVALKOV, Valeryi Konstantinovich**
Moscow, 119421 (RU)
• **BEREZIN, Kirill Andreevich**
Moscow, 117437 (RU)

(74) Vertreter: **Jeck, Anton**
**Jeck, Fleck & Partner mbB**
**Patentanwälte**
**Klingengasse 2**
**71665 Vaihingen/Enz (DE)**

(56) Entgegenhaltungen:
DE-T2- 3 884 136      RU-C1- 2 366 360
RU-C1- 2 617 270      RU-U1- 73 205
US-A1- 2002 087 201      US-A1- 2007 179 584

• **EMRAN SHEKH ET AL: "Concentric Ring Probe for Bioimpedance Spectroscopic Measurements: Design and Ex Vivo Feasibility Testing on Pork Oral Tissues", SENSORS, vol. 18, no. 10, 10 October 2018 (2018-10-10), CH, pages 3378, XP055933692, ISSN: 1424-8220, DOI: 10.3390/ s18103378**

EP 3 881 753 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 881 753 B1

**Beschreibung**

Die vorgeschlagene Erfindung bezieht sich auf das Gebiet der Erzeugung eines elektromagnetischen Feldes und der Messung der Eigenschaften eines elektromagnetischen Feldes zur Verwendung bei der Diagnose biologischer Gewebe, Organe und des Organismus insgesamt.

Aus dem Stand der Technik sind verschiedene Vorrichtungen und Verfahren bekannt, unter Verwendung einer Diagnose aufgrund der Messungen der Eigenschaften eines elektromagnetischen Feldes.

Bekannt ist ein Verfahren der Korneometrie und eine Vorrichtung zur Beurteilung des Zustandes der Haut (Epidermis). Dafür wird ein elektromagnetisches Wechselfeld mit der Frequenz von 0,9-1,2 MHz und ein speziell geformter Kondensator verwendet. Dies ermöglicht die Erzeugung eines elektromagnetischen Feldes in der Tiefe von 10-20 nm, hauptsächlich in der Hornschicht der Haut (Timofeev GA Methoden der apparativen Untersuchung der menschlichen Haut // Kosmetik und Medizin. 2005. Heft 4. S. 28-36). Ein Nachteil der Vorrichtung ist eine hohe Ausstrahlungsfrequenz und die Unmöglichkeit, innere Organe zu untersuchen.

Bekannt ist eine Vorrichtung der Korneometrie, basierend auf der Verwendung der Mikrowellenausstrahlung (40 GHz) (Korolkova T. N., Matytsin V. O., Turkovsky I. I., Kharin V. N. Perspektiven der Untersuchung des Wasseraustausches der menschlichen Haut mit der Methode der EHF-Dielektrometrie. Experimentelle und klinische Dermatokosmetologie 2003; 1(1):17-192). Ein Nachteil ist die Verwendung ultrahoher Frequenzen der elektromagnetischen Ausstrahlung.

Bekannt sind auch Vorrichtungen der Ultraschalluntersuchungen (USU), die in der Medizin sehr weit verbreitet sind (Strahlendiagnostik: Lehrbuch T. 1. / Herausgegeben von G.E. Trufanov - M., GEOTAR-Media, 2009). Der Nutzfrequenzbereich liegt innerhalb von 1-50 MHz. Ein Nachteil dieser Vorrichtungen ist die Notwendigkeit, den Luftspalt (Abstand) zwischen dem Sensor und biologischem Gewebe mit einem Gel zu beheben (wegzuschaffen). Die Anwendung des Gels erzeugt eine übermäßige Kühlung und ist bei der Untersuchung von Kindern und Neugeborenen unerwünscht. Ein Nachteil von Ultraschallvorrichtungen ist auch die Gefahr einer thermischen Schädigung des untersuchbaren Organs, insbesondere bei der Doppler-Ultraschalluntersuchung. Die thermische Erwärmung kann mehrere Grad erreichen, daher wird empfohlen, den Sensor der Ultraschallvorrichtung ständig zu bewegen, und die Ultraschalluntersuchung eines Fetus schwangerer Frauen wird eine begrenzte Anzahl von Malen durchgeführt, normalerweise nicht mehr als dreimal. Fragen zur Sicherheit der Ultraschalluntersuchung werden in Fachkreisen ständig besprochen.

Im RF-Patent No. 2112416 ist die Kontrolle des Zustands von Geweben und Organen in der postoperativen Phase beschrieben. Laut dieser Kontrolle werden die Impedanz und ihre Kapazitäten der Komponente der Sensorsonde bei mehreren Frequenzen gemessen, die direkt im Bereich der möglichen Pathologie angeordnet ist. Danach werden gemessene Werte mit den angegebenen Normativen verglichen. Gegebenenfalls werden die Messungen jederzeit wiederholt, um die Dynamik des Prozesses zu untersuchen. Der Sensor zur Durchführung des Verfahrens enthält ein Messelement mit einer Elektrode in Form von zwei halbzylindrischen leitfähigen Platten. Sie bedecken ein Dielektrikum aus einem Material mit niedriger Dielektrizitätskonstante. Das Messelement ist innerhalb des biologischen Neutralrohres angeordnet und nach dem Vier-Klemmen-Schaltschema zum Impedanzmesser angeschlossen, mit der Möglichkeit der Messung in einem bestimmten Frequenzbereich. Die Erfindung ermöglicht es, den Zustand eines Organs oder Gewebes dynamisch zu kontrollieren. Die Nachteile sind die Unbestimmtheit des Frequenzbereichs, die Abhängigkeit der Messergebnisse von der Position der leitfähigen Elektrodenbeplattungen des Messelements bei seiner Anordnung in der Nähe des untersuchbaren Organs. Offensichtlich, wenn die Elektrodenbeplattungen symmetrisch zum untersuchbaren Organ angeordnet sind, dann ist es eine Indikation, und wenn die Elektrode nur mit einer Beplattung zum Organ gedreht ist, dann ist es absolut andere Indikation. Es entstehen die Ungenauigkeit und die Subjektivität bei den Messungen, wenn nicht das Vorhandensein und die Menge biologischer Flüssigkeit (z. B. Lymphe, Blut oder Eiter), sondern der Zustand des biologischen Gewebes oder Organs kontrolliert werden muss.

Es wird ein Elektroden-Zwei- oder Vier-Klemmen-Messschaltschema verwendet. Darin haben die Anschlussleitungen einen kapazitiven Widerstand, der mit dem kapazitiven Widerstand der Elektroden vergleichbar ist. Die Notwendigkeit, den Widerstand der Anschlussleitungen für die gewählte Messfrequenz zu kompensieren, verringert die Effizienz bei den Messungen.

Durch US 2002/087201 A1, durch DE 38 84 136 T2 und durch EMRAN SHEKI ET AL: "Concentric Ring Probe for Bioimpedance Spectroscopic Measurements: Design and ex vivio Feasability Testing on Pork Oral Tissues", SENSORS, Bd. 18, Nr. 10, 10. Oktober 2018, pp. 3378 ist jeweils ein Sender aus drei konzentrisch angeordneten, runden Platten bekannt, von denen die größte und eine mittlere Platte mit jeweils einem Loch ausgeführt sind. Im Loch der jeweils größeren Platte ist die jeweils nächstkleinere, runde Platte angeordnet. Die Platten sind sämtliche voneinander elektrisch isoliert.

Durch US 2007/179584 A1 sind Sender mit Elektroden zur neuralen Stimulation bekannt. Der Sender besteht aus mehreren auf oder in einem kreisrunden Trägermaterial oder Trägersubstrat angeordneten, plattenförmigen Elektroden, umfassend eine zentrale Elektrode sowie eine oder mehrere um die zentrale Elektrode herum angeordnete weitere Elektroden. Der gesamte Sender ist im Körper eines Patienten implantierbar. Zumindest auf der Seite des Substrats, auf der die Elektroden angeordnet sind, stehen die Elektroden in elektrischen Kontakt mit dem Patienten. Die Elektroden sind über zu einem Elektrodenkabel angeordnete Leitungsdrähte miteinander verbunden.

Das Ziel der vorgeschlagenen Erfindung besteht darin, den Einfluss angegebener negativer und subjektiver Faktoren zu beheben.

Zur Lösung der gestellten Aufgabe wird eine Vorrichtung zum Erzeugen eines elektromagnetischen Feldes und zum Messen seiner Absorption durch ein leitendes Medium vorgeschlagen, aufweisend strukturell, einschließlich elektrisch, miteinander verbunden einen Wechselspannungsgenerator, einen Sender des elektromagnetischen Feldes und Mittel zum Messen der Absorption eines elektromagnetischen Feldes. Wobei wird ein Sender (Strahler) verwendet, der die Erzeugung eines torodialen elektromagnetischen Feldes ermöglicht, aufweisend mindestens zwei runde Platten, die so ausgeführt und in einer Ebene angeordnet sind, dass eine Platte mindestens ein Loch (eine Öffnung) aufweist, wo mindestens eine andere Platte angeordnet ist. Wobei ist die Platte mit einem Loch von den anderen Platten elektrisch isoliert und das erzeugte elektromagnetische Feld ist in einer Ebene symmetrisch. Die Messung der Absorption des elektromagnetischen Feldes erfolgt dabei mit dem Programm der Fast Fourier-Transformation in einem Rechner mit der Bestimmung der Amplituden der Oberwellen, der Berechnung der Ausstrahlungsleistung und der Verteilung nach der Oberwellenleistung des Sendesignals. Dabei stellt der Wechselspannungsgenerator den Betrieb des Senders mit der Frequenz von weniger als 100 kHz sicher.

Es empfiehlt sich, dass die Parameter des Wechselspannungsgenerators konstant sind und die ausgestrahlte Leistung nur durch den Widerstand des Senders bestimmt wird. Das Anlegen der frequenzvariablen Spannung zum Sender vom Generator erfolgt in der Regel durch den Widerstand. Dies ermöglicht die Berechnung der Ausstrahlungsleistung, bei der bekannten Generatorspannung und gemessenen Spannung am Sender.

Der Sender kann mehr als zwei Platten enthalten, wobei eine Platte in ihrer Ebene mindestens ein Loch aufweist. Darin sind andere Platten angeordnet, wobei alle Platten miteinander elektrisch verbunden und von der Platte mit einem Loch isoliert sind.

Der Sender wird an den Wechselspannungsgenerator mit der Frequenz weniger als 100 kHz angeschlossen und über einen ADW und eine Kommunikationsschnittstelle werden die Signale zum Rechner abgegeben, insbesondere zum Smartphone, Computer oder Laptop.

Weiterhin ist eine Kommunikationsschnittstelle zur Signalabgabe vom Sender zu einem Rechner vorgesehen, insbesondere Smartphone, Computer oder Laptop, wo mit einem vorgegebenen Programm die mittlere Ausstrahlungsleistung gemessen und analysiert wird, unter Berücksichtigung der Absorption im Frequenzbereich 100 Hz - 20 kHz, sowie das Ausstrahlungsleistungsspektrum unter Berücksichtigung der Absorption gemessen wird.

Der Sender kann mehr als zwei Platten aufweisen, wobei eine Platte in ihrer Ebene mindestens ein Loch aufweist, darin sind andere Platten angeordnet, wobei alle Platten miteinander elektrisch verbunden und von der Platte mit einem Loch isoliert sind. Das Loch kann rund oder oval sein, bei mehreren Löchern sind sie symmetrisch. Der Sender wird an einen Wechselspannungsgenerator mit der Frequenz von weniger als 100 kHz angeschlossen.

Die Vorrichtung kann zur Messung der Parameter des untersuchbaren Areals im Ausstrahlungsbereich eines elektromagnetischen Feldes mit der Frequenz von weniger als 100 kHz verwendet werden, vorzugsweise weniger als 20 kHz. Dafür wird der vorgeschlagene Sender eines elektromagnetischen Feldes verwendet. Die Parameter des untersuchbaren Areals kann man nach der Fähigkeit bestimmen, das ausgestrahlte elektromagnetische Feld zu absorbieren. Dabei können Testmessungen von gesundem Gewebe und Organ als Referenzmessungen zur Analyse des Zustandes, der Abweichungen und der Erkennung von Pathologien verwendet werden.

In der vorgeschlagenen Vorrichtung wird ein elektromagnetisches Feld mit der Frequenz von weniger als 100 kHz, vorzugsweise weniger als 20 kHz, erzeugt und für Messungen verwendet. Der Sender erzeugt ein elektromagnetisches Feld und dann wird die Fähigkeit eines Gewebes oder Organs gemessen, dieses elektromagnetische Feld zu absorbieren. Durchgeführte Untersuchungen verschiedener Hautareale, Weichgewebe und innerer Organe im Vergleich zur Absorption des elektromagnetischen Feldes durch nicht-biologische Gewebe, z.B. Luft, Holz, Metall, Wasser, haben einen gewissen Unterschied gezeigt. Die Forschung bei der Frequenz 5-6 kHz und bei der Frequenz 10-13 kHz durch die Spektralanalyse der absorbierten Leistung im Bereich von 10-20 kHz hat das Vorhandensein anderer Frequenzen im Frequenzspektrum neben der Hauptausstrahlungsfrequenz gezeigt, abhängig vom Zustand des untersuchbaren Areals und dem Vorhandensein darin irgendwelcher Abweichungen oder Pathologien.

Es ist bekannt, dass man heute nach Pulsarrhythmie, elektrischen Signalen bei der Messung des Herzkardiogramms dank einem großen statistischen Material das Vorhandensein der einen oder anderen Abweichung von der Norm feststellen kann.

Es ist ganz offensichtlich, dass man nach dem Spektrum der Absorptionsfrequenzen eines elektromagnetischen Feldes Abweichungen von der Norm feststellen kann, beim Vorhandensein statistischer Daten, entsprechender Kalibrierung, Auswertung (Interpretation) und Analyse. Somit ist die Standardisierung (Normierung) und Kalibrierung (Eichung) von Messwerten für die Zustandsanalyse ein Standardverfahren.

Es ist bekannt, dass elektromagnetische Schwingungen sowohl in der Nahzone als auch in der Fernzone existieren und ein elektromagnetisches Feld kann in großer Entfernung vom Sender existieren. Eine elektromagnetische Welle ist eine Art des elektromagnetischen Feldes, wo sich die Stärke des elektrischen und magnetischen Feldes umgekehrt proportional zum Abstand vom Sender ändert. In der Nahzone werden die sogenannten "stehenden" Wellen beobachtet und die Phase der Schwingungen des elektrischen Feldes unterscheidet sich von der Phase der Schwingungen des magnetischen Feldes um π/2. In der Fernzone fallen die Phasen der Schwingungen des elektrischen Feldes und des magnetischen Feldes zusammen. (Lavrov VM Theorie des elektromagnetischen Feldes und die Grundlagen der Funkwellenausbreitung. Lehrbuch für Universitäten für Kommunikation. Verlag "SVYAZ". Moskau. 1964 S.231-233). Das elektromagnetische Feld ist kontinuierlich, aber die Stärke des elektromagnetischen Feldes ändert sich in der Nahzone umgekehrt proportional zur Kubikzahl der Entfernung für eine elektrische Komponente der elektromagnetischen Schwingungen und umgekehrt proportional zum Quadrat der Entfernung (vom Sender) für eine magnetische Komponente elektromagnetischer Schwingungen. Und in der Fernzone ändert sich die Stärke des elektromagnetischen Feldes umgekehrt proportional zur Entfernung vom Sender. (Bessonov L.A. Theoretische Grundlagen der Elektrotechnik: Elektromagnetisches Feld. Lehrbuch für Universitätsstudenten. -7. Auflage, M.: Higher School, 1978. S. 174-175; Tamm I.E. Grundlagen der Elektrizitätslehre: Lehrbuch für Universitäten. - 11. Hrsg., M.: FIZMAT LIT. 2003, S. 468-470).

Es ist bekannt, dass für Frequenzen unter 100 kHz die Wellenlänge im Vakuum mehr als 3 km beträgt, während biologische Gewebe und Organe ein leitendes Medium sind. Die elektrische Leitfähigkeit biologischer Gewebe und Organe ist unterschiedlich und liegt im Bereich von 0,06-1,5 S/m (Simmens/Meter) (1/Ohm m), siehe L.I. Kalakutskij Grundlagen der Impedanzmessung biologischer Gewebe [Elektronische Ressource]: Elektron, Lehrbuch, Handbuch / LI Kalakutskiy, SA Akulov, AA Fedotov; Ministerium für Bildung und Wissenschaft Russlands, Samar. Staats- Luft- und Raumfahrt un-t sie. S. P. Koroleva (nationale Forschungsuniversität). - Elektron, Text und Grafik. Dan. - Samara, 2011 S. 9.

Für leitendes Medium versteht man unter Wellenlänge die Strecke entlang der Ausbreitung der Welle, bei der sich die Phase der Schwingung um 2π ändert. Die Wellenlänge wird aus der Gleichung bestimmt:

$$\lambda k = 2\pi, \text{ wo}$$

$$k = \sqrt{(\omega\gamma\mu)}/2$$

$\gamma$ - Leitfähigkeit des Mediums

$\mu$ - magnetische Permeabilität

Siehe Bessonov L.A. Theoretische Grundlagen der Elektrotechnik: Elektromagnetisches Feld. Ein Lehrbuch für Universitätsstudenten. -7. Auflage, M.: Hochschule, 1978. S. 138-139.

Es wurde experimentell festgestellt, dass für die Frequenz von 5 kHz und $\gamma \approx 0{,}2 C_M/_M$ (Leber, Haut, Muskeln), $\mu \approx 1{,}26 \times 10^{-6}$ (magnetische Permeabilität) $\lambda \approx 100$ m ist. Für die Frequenz von 100 kHz ist $\lambda \approx 25$ m. Für biologische Gewebe und Organe liegt die Wellenlänge also bei Frequenzen unter 100 kHz im Bereich von ca. 25-100 m.

Nach der Theorie des elektromagnetischen Feldes kann die Energieausstrahlung durch einen elektrischen Dipol mit der Länge $\ell \ll \lambda$. erfolgen. Ohne auf die Theorie einzugehen, ist es offensichtlich, dass das elektromagnetische Feld in der Nähe des Senders (Oszillators) keine Kugel- (sphärische) oder ebene (Wellen-) elektromagnetische Welle ist, sondern hat eine komplexere Form, da sich die Stärke des elektrischen Feldes umgekehrt proportional zur Kubikzahl der Entfernung ändert, und die Stärke des magnetischen Feldes ist umgekehrt proportional zum Quadrat der Entfernung vom Sender. Wobei finden in der Nahzone zwei Prozesse statt. Der erste Prozess ist der Prozess eines periodischen Energieaustausches zwischen der Energiequelle, an die der Sender angeschlossen ist, und der Nahzone. Die Energie wird bald der Quelle entnommen und im elektromagnetischen Feld der Nahzone angesammelt bald wieder an die Quelle abgegeben. Der zweite Prozess ist der Prozess einer Energieausstrahlung. Er charakterisiert den Wellenprozess in der Nahzone. Die ausgestrahlte Energie ist relativ gering im Vergleich zu der Energie, die periodisch im elektromagnetischen Feld der Nahzone angesammelt und dann an die Stromquelle abgegeben wird. Siehe Bessonov L.A. Theoretische Grundlagen der Elektrotechnik: Elektromagnetisches Feld. Ein Lehrbuch für Universitätsstudenten. -7. Auflage, M.: Hochschule, 1978. S.175.

Die vorgeschlagene Erfindung basiert auf der Möglichkeit, die Absorption durch verschiedene Medien (biologische und leitende) des elektromagnetischen Feldes niedriger Frequenz in der Nahzone zu messen.

Aus dem Patent der Russischen Föderation Nr. 2287891 ist beispielsweise ein Detektor der amplitudenmodulierten Schwingungen bekannt, der auf einem Ferritring in Form von quadratischem Querschnitt ausgeführt ist. Auf einer Hälfte sind auf jeder seiner Flächen Bandelektroden angeordnet und auf einer anderen Hälfte ist eine Solenoidwicklung angeordnet. Wobei ist die amplitudenmodulierte Schwingung an die Eingänge des ersten und zweiten Resonanzverstärkers durch phasenverschiebende jeweils um +45° bzw. -45° Ketten angelegt.

Der Ausgang des ersten Resonanzverstärkers ist zu einem Paar am Ferritring gegenüberangeordneter Bandelektroden angeschlossen. Der Ausgang des zweiten Resonanzverstärkers ist zu einem anderen Paar am Ferritring gegenüberangeordneter Bandelektroden angeschlossen. Und die lineare gleichgerichtete Ausgangsschwingung wird in der Solenoidwicklung angefacht. Man kann einen dreieckigen toroidalen ferritischen Magnetleiter mit einer Wicklung und drei Elektroden verwenden, um diese an eine dreiphasige AM-Signalquelle anzuschließen. Unter Verwendung der Ferritringe mit einem nicht quadratischen, sondern rechteckigen Querschnitt ist es möglich, ein rotierendes elektrisches Feld mit einer entsprechenden Einstellung der Verstärkungskoeffizienten $K_1$ und $K_2$ im ersten und zweiten Resonanzverstärker zu erhalten, so dass die gleiche Stärke des elektrischen Feldes im Ferritring für die entsprechenden Bandelektrodenpaare sichergestellt wird. Unter Einwirkung eines zirkular polarisierten (meist elliptischen) elektrischen Feldes statt eines runden gibt es auch eine Magnetisierung des Ferritringes, jedoch mit einem kleineren Wert. Dies ermöglicht eine Variation der Größen der Phasenverschiebungen in den Phasenverschiebungsketten.

Aus dem RF-Patent Nr. 2617270 ist wiederum eine Spule zur Visualisierung durch Magnetinduktionstomographieverfahren bekannt. Sie weist mehrere erste und mehrere zweite konzentrische leitende Windungen auf, die jeweils in der ersten und zweiten Ebene angeordnet sind. Wobei ist die zweite Ebene von der ersten um den Abstand zwischen den Ebenen getrennt. Wobei sind mehrere erste Windungen mit mehreren zweiten Windungen seriengeschaltet. Das System zur Visualisierung durch Magnetinduktionstomographieverfahren weist eine Radiofrequenz -Energiequelle, eine mit der Radiofrequenz -Energiequelle verbundene Spule und ein Messschaltschema auf. Die Verwendung von Erfindungen wird eine Visualisierung durch Magnetinduktionstomographieverfahren unter Verwendung einer einzelnen Spule ermöglichen.

Als nächstliegende Analogielösung kann die Vorrichtung gemäß dem RF-Patent Nr. 2366360 angesehen werden Die Erfindung ist zum Messen der Impedanz von biologischen Geweben bestimmt. Die Vorrichtung weist einen Sinusgenerator mit einstellbarer Ausgangssignalfrequenz, einen elektronischen Schalter, einen Messblock, Messelektroden, einen analogen Multiplexer, einen Breitbandverstärker, einen Mittelwertwandler mit einstellbarer Zeitkonstante, einen Analog-Digital Wandler (ADW), ein transversales digitales Filter, einen Block zur Steuerung und Informationsverarbeitung und einen Indikator auf. Die Erfindung stellt die Erweiterung des Frequenzbereichs und des Arbeitsbereichs der Messimpedanzwerte sicher, sowie die Erhöhung der Genauigkeit, die Möglichkeit, spezifische Impedanzwerte zu messen, eine statistische Verarbeitung und Berechnung verschiedener Funktionsparameter, die Ausgabe der Ergebnisse an den Indikator. Allerdings weist auch diese Vorrichtung die oben genannten Nachteile auf.

Es zeigen:

Fig. 1     ein allgemeines Funktionsdiagramm der Vorrichtung;

Fig. 2     Beispiele der Form von Sendern der Flachelektroden zur Ausstrahlung eines elektromagnetischen Feldes;

Fig. 3     das elektrische Ersatzschaltbild einer Zelle;

Fig. 4     die Abhängigkeit der Dielektrizitätskonstante eines Skelettmuskels (durchgezogene Linie) und 0,85 % NaC1-Lösung in Wasser (Strichlinie) von der Frequenz des elektrischen Feldes;

Fig. 5     ein Messdiagramm für die erste Kuhherde;

Fig. 6     ein Messdiagramm für die zweite Kuhherde

Fig. 7     ein Diagramm des Spektrums und der Ausstrahlungsleistung der Vorrichtung unter Verwendung eines Spektrum- und Signalanalysators R&S FSV (Roshde & Schwarz) und einer aktiven Stabantenne zur Messung der Stärke des elektrischen Feldes R&S HFH2-Z6E (Roshde & Schwarz) in der Entfernung von 1 cm vom Sender der Vorrichtung.

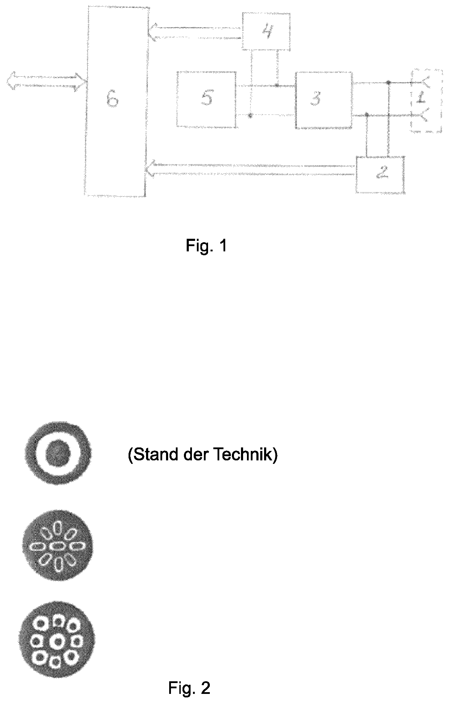

Das Funktionsschema der Vorrichtung auf der Fig. 1 enthält folgende Elemente: 1 - Sender, 2 - ADW (Analog-Digital-Wandler), 3 - Ausstrahlungsspannungsformer, Generator f= 6 kHz, 4 - ADW, 5 - Referenzfrequenzgenerator, 6 - USB-Kanal-Controller.

An die Klemmen des Senders (1) wird ein Ausstrahlungsspannungsgenerator (3) mit der Frequenz von $6 \pm 1$ kHz angeschlossen. Zwischen den Platten (Elektroden) fließt ein elektrischer Wechselstrom. Ein Dielektrikum (z.B. Luft) umgibt die Elektroden, was die Verschiebungsstromerzeugung sicherstellt. Der umgebende Raum hat Stromleitfähigkeit, was die Leitungsstromerzeugung sicherstellt. Um die Linien mit dem Wechselstrom herum wird ein magnetisches Wechselfeld gebildet. Der Ausstrahlungsspannungsformer (3) hat einen eingebauten Widerstand zur Begrenzung der Ausstrahlungsleistung. Durch den programmierten ADW (2) werden mit einem Rechner Spannungen gemessen und die Leistung und der Leitfähigkeitswert berechnet. Zuerst wird die Ausstrahlungsleistung in den freien Raum gemessen und dann beim Kontakt mit einem biologischen Objekt. Die Ausstrahlungsleistung wird gemessen, mit welcher der Sender innerhalb einer Schwingungsperiode mit dem umgebenden Raum austauscht. Bei bekannter Spannung des Referenzfrequenzgenerators (5) und gegebenem Widerstand des Ausstrahlungsspannungsformers (3) kann man den Widerstand des Senders (1) berechnen, d.h. die Leitfähigkeit, den Kehrwert des Widerstands. In diesem Fall wird angenommen, dass diese Leitfähigkeit des Senders durch die Leitfähigkeit des biologischen Objekts bestimmt wird. Wenn die Größe des Senders erheblich geringer ist als die Wellenlänge des elektromagnetischen Ausstrahlungsfeldes, ist sein Widerstand praktisch null gleich.

$$P=V^2/(R(3) + R \text{ biolog. Objekt})$$

Mit dieser Formel wird die Ausstrahlungsleistung (Absorptionsleistung) für jede Ausstrahlungsoberwelle und die Gesamtausstrahlungsleistung für die ersten drei Oberwellen berechnet.

Es ist zu beachten, dass im Gegensatz zur radioaktiven Ausstrahlung die Energie eines niederfrequenten elektromagnetischen Feldes, weniger als 100 kHz, im menschlichen Körper nicht gestreut wird, sondern nur innerhalb einer Schwingungsperiode des elektromagnetischen Feldes an der Ansammlung und Rückführung von Energie zum Sender teilnimmt. Die vorgeschlagene Vorrichtung misst die Leistung der Energie, mit welcher der Sender mit dem untersuchbaren Bereich austauscht. Ihr Wert hängt von der Leitfähigkeit des biologischen Objekts für die gegebene Frequenz ab. Daher wird die Leitfähigkeit des untersuchbaren Bereichs berechnet. Der Rechner für Berechnungen, beispielsweise über den USB-Kanal, erhält kontinuierlich die Messergebnisse der Spannung am Sender und am Eingang des Wechselspannungsgenerators. Die Messung erfolgt über eine eingebaute Mikroschaltung des 2-Kanal-ADWs mit einem eingebauten USB-Kanal-Controller. Ebenfalls wird im Rechner zur Berechnung der Widerstandswert eingetragen, über den die Wechselspannung vom Generator zum Sender zugeführt wird. Das Standardprogramm des Signal-Spektrum-Analysators ermöglicht durch Fast Fourier Transformation in Echtzeit die Werte der Oberwellen auf dem Bildschirm zu beobachten, in diesem Fall im Frequenzbereich 100 Hz - 20 kHz, da die konkrete CD-Vorrichtung einen Spannungsgenerator mit der Frequenz von 6 ± 1 kHz hat. In der Nahzone ist dies ein Prozess des periodischen Energieaustausches zwischen der Energiequelle, an die der Sender angeschlossen ist, und der Nahzone. Die Energie wird bald der Quelle entnommen und im elektromagnetischen Feld der Nahzone angesammelt bald wieder an die Quelle abgegeben. Dafür gibt es zwei Erklärungen. Ether-Anhänger glauben, dass Ether diejenige Essenz ist, welche die Energie ansammeln und abgeben kann. Was wird die Energie ansammeln und abgeben, wenn kein Ether vorhanden ist? Ether-Gegner behaupten, dass im Dielektrikum die sogenannten Verschiebungsströme entstehen, die eigentlich Ladungen am Sender bilden. Ein magnetisches Wechselfeld entsteht, als würde ein Strom fließen. Aber egal wie der Mechanismus der Ansammlung und des Energieaustausches zwischen dem umgebenden Raum und dem Sender erklärt wird, bleibt die Tatsache, dass die Größe der Energie des elektromagnetischen Feldes von den Parametern des umgebenden Raums der Nahzone abhängt. Eigentlich lässt die Vorrichtung die Parameter des umgebenden Raums messen.

Beispiele für Sender aus Flachelektroden zur Ausstrahlung eines elektromagnetischen Feldes sind auf der Fig. 2 dargestellt.

Die vorgeschlagene Vorrichtung mit einem Sender und einer Messvorrichtung arbeitet wie folgt.

Um ein elektromagnetisches Feld zu erzeugen, wird ein Sender verwendet, der aus zwei Platten besteht. Sie sind in derselben Ebene so angeordnet, dass eine Platte ein Loch hat, in dem eine andere Platte angeordnet ist. Die Platten sind voneinander und von dem untersuchbaren Bereich elektrisch isoliert. Der Sender kann aus mehreren Platten bestehen, aber mindestens eine Platte muss in ihrer Ebene Löcher haben, in denen andere Platten angeordnet sind. Alle sind elektrisch miteinander verbunden und von der Platte mit Löchern isoliert. Die Platten sind an den Wechselspannungsgenerator mit der Frequenz von weniger als 100 kHz angeschlossen und durch den ADW und die Kommunikationsschnittstelle werden die Signale zum Rechner, insbesondere zum Smartphone, Computer oder Laptop zugeführt. Da wird durch das vorgegebene Programm die durchschnittliche Strahlungs-(Absorptions-)Leistung im Frequenzbereich 100 Hz - 20 kHz gemessen und analysiert und das Ausstrahlungs-(Absorptions-)Leistungsspektrum wird gemessen, mit der Möglichkeit, spektrale Eigenschaften der sichtbaren Spitzen im Spektrum zu bestimmen.

An den Sender wird die Spannung von dem Wechselstromgenerator durch den Widerstand angelegt (um den fließenden Strom zu messen). Durch den eingebauten ADW wird die Spannung am Sender gemessen und über die USB-Schnittstelle wird dieser Wert an ein Smartphone, einen Computer oder Laptop gesendet. Da sich die Spannung an die Sender von der Sinusform unterscheidet, werden mit dem Fast Fourier Transformation (FFT)-Programm die Amplituden der Oberwellen bestimmt und die Strahlungsleistung wird berechnet. Im Programmmodus "Spektrumanalysator" kann man die Verteilung nach der Leistung der Oberwellen des Sendesignals beobachten. Die Parameter des Wechselspannungsgenerators sind konstant und die ausgestrahlte Leistung wird nur durch den Widerstand des Senders bestimmt. Der Widerstand des Senders hängt von den konstruktiven Parametern des Senders und von der elektrischen Leitfähigkeit des Mediums ($\gamma$), seiner dielektrischen ($\varepsilon$) und der magnetischen Permeabilität ($\mu$) ab.

Wellenwiderstand des Mediums (GOST R 52002-2003 S. 42):

$$Z \text{ Wellen} = \sqrt{i\omega\mu\mu o / (\gamma + i\omega\varepsilon\varepsilon o)}$$

Siehe Bessonov L.A. Theoretische Grundlagen der Elektrotechnik: Elektromagnetisches Feld. Ein Lehrbuch für Universitätsstudenten. -7. Auflage, M.: Hochschule, 1978. S. 128-139

In stark leitfähigen Medien wie Metallen überwiegt der Wert $\gamma$. In biologischen Geweben ist der Wert $\gamma$. nicht groß und der Wellenwiderstand wird durch den Wert der Dielektrizitätskonstante $\varepsilon$ bestimmt.

Für das Luftmedium Z Wellen $= \sqrt{\mu o / \varepsilon o}$ =377 Ohm. (Siehe Bessonov LA Theoretische Grundlagen der Elektrotechnik: Elektromagnetisches Feld. Lehrbuch für Universitätsstudenten. -7. Auflage, M.: Hochschule, 1978. S.150).

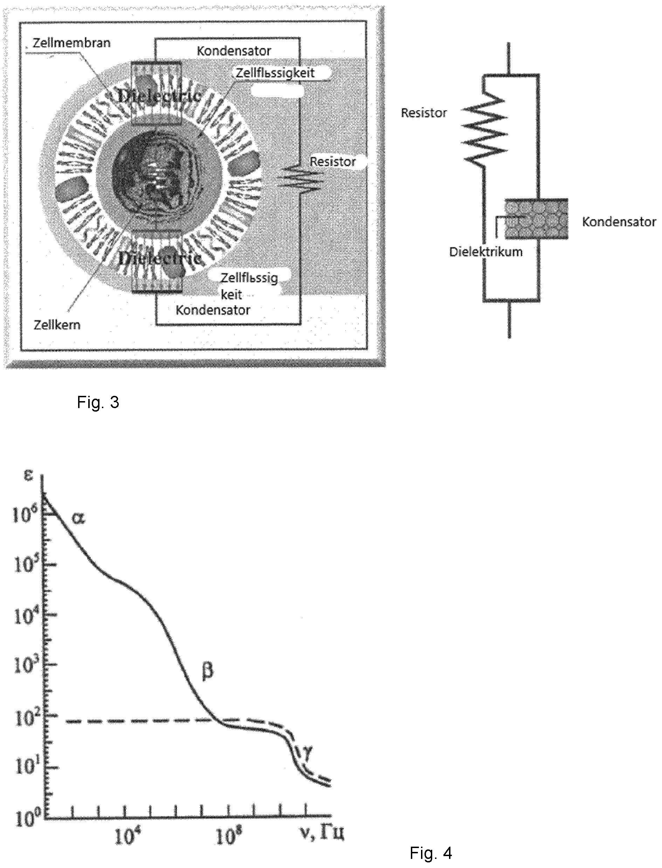

Fig. 3 zeigt das elektrische Ersatzschaltbild einer Zelle, siehe LI Kalakutskiy. Grundlagen der Impuls-Impedanzmessung biologischer Gewebe. FBGOU VPO Samara State University benannt nach Akademiemitglied S.P. Korolev "S. 67.

Fig. 4 zeigt die Abhängigkeit der Dielektrizitätskonstante eines Skelettmuskels (durchgezogene Linie) und 0,85 % NaC1-Lösung im Wasser (Strichlinie) von der Frequenz des elektrischen Feldes, siehe B.I. Sedunov, D. A. Frank-Kamenetsky. Dielektrizitätskonstante biologischer Objekte. Fortschritte in den physikalischen Wissenschaften. 1963 April. T. LXXIX, Ausg. 4.

Die Dielektrizitätskonstante einer Zelle hängt vom Zustand der Zellmembran ab und die Leitfähigkeit des Mediums wird durch die Extrazellularflüssigkeit bestimmt. Die Dielektrizitätskonstante von Blut beträgt $10^2$ der Zelle bis zu $10^6$.

Dies bedeutet eine große Abhängigkeit des Widerstands des Mediums vom Zustand der Zellen bei dieser Messmethode, daher misst man die Gesamtäquivalentleitfähigkeit (den Kehrwert des Widerstands).

Der Sender wird zum untersuchbaren Bereich angelegt und die spektralen Eigenschaften des elektromagnetischen Feldes sowie unter Berücksichtigung seiner Absorption der Leitfähigkeitswert fixiert. Nach dem Auftreten der Abweichungen von den angegebenen Eigenschaften des Spektrums und der Leitfähigkeit des Normalzustands wird das Vorhandensein einer Abweichung festgestellt, was die Folge einer Pathologie sein kann.

Derzeit sind mehrere Versuchsmuster der Cyber-Doctor-Vorrichtung (CD-Vorrichtung) hergestellt. Die CD-Vorrichtung wird über einen USB-Port zum Computer, Smartphone oder Laptop angeschlossen. Diese Vorrichtung ersetzt weder Ultraschall noch MRT, aber mit einer einfachen Messung ermöglicht die Frage schnell zu beantworten, ob es einen Verdacht auf eine Abweichung von der angegebenen Norm in einem Gewebe oder Organ gibt. Im Falle einer bekannten Pathologie ist beispielsweise eine ernsthafte Analyse des Zustandes des Patienten erforderlich, um die Dynamik der Entwicklung der Pathologie zu bestimmen. Mit der vorgeschlagenen Vorrichtung kann man die Dynamik ohne Verwendung der körperschädigenden Strahlung und Forschungsmethoden verfolgen, um den Zustand zu kontrollieren und das Risiko einer Abweichung und die Möglichkeit einer Pathologie einzuschätzen und gegebenenfalls eine Entscheidung über schwerere Untersuchungen zu treffen. Mit der CD-Vorrichtung kann man beispielsweise den Hautzustand auch nach kosmetischen Behandlungen kontrollieren, um den Wirkungsgrad und Folgen zu bestimmen. Es ist wichtig zu bemerken, dass es sich um eine sichere Untersuchung handelt, die regelmäßig und sogar selbständig durchgeführt werden kann, um den Zustand und die Dynamik bei Bedarf oder zu Präventionszwecken zu kontrollieren. In diesem Sinne erfüllt die Vorrichtung präventive Untersuchungsfunktionen, ersetzt jedoch die medizinischen Untersuchungen für die Verwendung in der medizinischen Praxis nicht.

Es wird eine Senderantenne verwendet, die vom Patienten elektrisch isoliert ist. Die Zufuhr der Wechselspannung zum Sender vom Generator erfolgt über den Widerstand, was die Berechnung der Ausstrahlungsleistung ermöglicht, bei bekannter Generatorspannung und gemessener Spannung am Sender. Die bequemste Form von Platten und Löchern ist rund oder elliptisch, jedoch können die Löcher eine beliebige Form haben.

Der minimale Abstand mit der Platte (n) im Loch wird nur durch die Möglichkeiten der Herstellungstechnologie bestimmt, und der maximale Abstand reduziert nur die Fläche der Senderplatte. Die Fläche der Senderplatte sollte deutlich größer sein als die Fläche der Leiter, über welche der Sender an den Wechselspannungsgenerator angeschlossen ist).

Die Vorrichtung muss eine gute Ergonomie aufweisen, damit die Vorrichtung bequem zu halten ist und beim Bewegen über den Körper des Patienten keine Missempfindungen durch mögliche Reibung entstehen, d.h. keine scharfen Ecken. Das Schnittstellenkabel muss lang genug und flexibel sein, um es an einen Computer anzuschließen.

Die bekannten Analogielösungen des Senders sind Platten, die in derselben Ebene angeordnet sind. Die Platten sind rund oder quadratisch sowie in Form von zwei Kämmen, die in den Abständen gegenseitig angeordnet sind.

Als Material für die Platten können folienkaschiertes Glashartgewebe und Kupferfolie verwendet werden.

Die Messung wird wie folgt durchgeführt: vom Wechselspannungsgenerator zum Sender wird über den Widerstand Spannung zugeführt; durch den Analog-Digital-Wandler (ADW) werden die gemessenen Spannungen am Sender und am Wechselspannungsgenerator (die Vorrichtung enthält eine zweikanalige ADW-Mikroschaltung mit USB-Schnittstellencontroller) in digitale Form umgewandelt und über die USB-Schnittstelle an einen Computer übertragen (Smartphone, Laptop etc.); mit einem Standard-Fast-Fourier-Transformation-(FFT)-Programm wird die Amplitude der Oberwellen bestimmt und die Ausstrahlungsleistung wird berechnet. Danach wird die Leitfähigkeit des biologischen Objekts berechnet. In der Nahzone findet nur der Energieaustausch zwischen der Umgebung und dem Sender statt. Das elektromagnetische Feld wird innerhalb der Nahzone erzeugt und es gibt keine Ausstrahlung der elektromagnetischen Wellen außerhalb der Nahzone. In der Radiotechnik wird die Energie des elektromagnetischen Feldes in der Nahzone als reaktive Energie bezeichnet. Elektromagnetische Wellen in der Nahzone werden als "stehende Welle" bezeichnet, da die in der Nahzone auftretende elektromagnetische Welle nicht über die Nahzone hinausgeht. In der Nahzone wird die Energie der elektromagnetischen Ausstrahlung von der Umgebung absorbiert (angesammelt) und dann innerhalb einer Schwingungsperiode an den Sender zurückgegeben. Absorbiert (angesammelt) bedeutet nicht gestreut, d.h. in Wärme- oder eine andere Energieform umgewandelt.

Zum Sender wird über den Widerstand von dem Wechselspannungsgenerator Spannung zugeführt. Wenn die Größe des Senders erheblich geringer ist als die Wellenlänge des elektromagnetischen Strahlungsfeldes, ist sein Widerstand praktisch null gleich. Der Wellenwiderstand der Umgebung hängt wiederum von der dielektrischen und magnetischen Permeabilität und Leitfähigkeit des Mediums ab: $P=V^2/(R_D + R$ biologisches Objekt), wo $R_D$ der zusätzliche Widerstand ist (zwischen dem Sender und dem Wechselspannungsgenerator). $P= V \times I$, bei dem bekannten $R_D$ ist es einfach, R biologischen Objekt zu bestimmen. Unter Verwendung der obigen Formel werden die Absorptionsausstrahlungsleistung für jede Ausstrahlungsoberwelle und die Gesamtausstrahlungsleistung für die ersten drei Oberwellen berechnet. Die Absorptionsleistung des elektromagnetischen Feldes ist diejenige Leistung, die der Sender mit einem biologischen Objekt in der Nahzone austauscht. Da die Abmessungen des Senders im Vergleich zur Wellenlänge des elektromagnetischen Strahlungsfeldes gering sind, geht die Strahlung über die Nahzone nicht hinaus. Es erfolgt nur die Ausstrahlung in die Nahzone (zu einem biologischen Objekt) und dann zurück zum Sender, der Energieaustausch innerhalb einer Schwingungsperiode des elektromagnetischen Feldes.

Die Berechnung der spektralen Eigenschaften und deren Fixierung (Visualisierung) erfolgt durch den Rechner und wird auf dem Bildschirm angezeigt. Ein Smartphone, ein Laptop- das sind Rechner, in denen durch Standardprogramme der Fast Fourier Transformation (FFT) die spektralen Eigenschaften der Messgrößen berechnet werden. Sie werden zunächst durch den ADW in digitale Werte umgewandelt und über die USB-Schnittstelle an den Rechner übertragen.

Die Wellenlänge wird durch Umgebungsparameter wie Dielektrizitätskonstante, magnetische Permeabilität und Leitfähigkeit (bei leitfähigen Medien) bestimmt. In diesem Fall beträgt die Wellenlänge für biologische Körper $\lambda \approx 25$ m. Ausgehend davon, dass die Körpergröße einer Kuh etwa 2 Meter und die Größe des Senders etwa 2 cm beträgt, auch unter Berücksichtigung einer Stabantenne (die Größe ist in der Beschreibung angegeben), beträgt die Länge des Senders für Messzwecke nicht mehr als 10 cm.

Die in der Nahzone ablaufenden Prozesse sind durch den Energieaustausch zwischen der Umgebung und dem Sender (Antenne) innerhalb einer Schwingungsperiode gekennzeichnet. Die Kuh (oder der menschliche Körper) stellt eine bedeutende Umgebung für einen so kleinen Sender dar.

Unter Absorption ist die Leistung der Energie zu verstehen, welche der Sender innerhalb einer Schwingungsperiode des elektromagnetischen Feldes mit der Umgebung austauscht. Die absorbierte Ausstrahlung kann vom Medium ganz oder teilweise wieder ausgestrahlt werden: bei Frequenzen, die sich von der Frequenz der absorbierten Ausstrahlung unterscheiden.

Die Abhängigkeit der Absorption von der Frequenz wird durch das Absorptionsspektrum des Stoffes bestimmt, und das Größenverhältnis des absorbierten Flusses zur Größe des einfallenden Flusses wird durch den Absorptionskoeffizienten bestimmt. Die quantitativen Eigenschaften des Absorptionsprozesses erforscht die Photometrie. Der inverse Absorptionsprozess ist die Streuung der elektromagnetischen Ausstrahlung, dessen Sonderfall die Reflexion elektromagnetischer Wellen an der Grenzfläche der Medien ist.

In lebenden biologischen Medien kann die Dielektrizitätskonstante (der Zellmembranen) den Wert von mehr als 1 Million erreichen, während die Dielektrizitätskonstante biologischer Flüssigkeiten etwa 80 Einheiten beträgt (siehe Fig. 3).

Die Energie, die gestreut wird und nicht zum Sender zurückkommt, ist auf die Leitfähigkeit biologischer Gewebe zurückzuführen und sie ist erheblich gering im Vergleich zur Reaktionskomponente der ausgestrahlten Energie für die Grundausstrahlungsfrequenz bei Größenordnung von 6 kHz und deren Oberwellen innerhalb von 20 kHz und sogar 100 kHz.

Zur Nasenrachenhöhlen- und Zervixuntersuchung weist die Innenplatte des Senders einen Stiftsender mit dem Durchmesser von weniger als 4 mm und der Länge von 15 bis 100 mm auf.

Zu Versuchszwecken wurden mit der CD-Vorrichtung Kälber und zwei Kuhherden mit je 200 Stück untersucht. Die Kälber wurden von Mitarbeitern des Allrussischen Forschungsinstituts für experimentelle Veterinärmedizin, benannt nach K.I. Skrjabin und Ya.R. Kovalenko unter der Leitung vom Akademiemitglied I.M. Gulyukin in der Versuchswirtschaft des Instituts im Dorf Konobeevo im Gebiet Moskau untersucht. Die Untersuchung der Kühe wurde auf dem Bauernhof Alekseevskoe der JSC Moskvoretsky Sovkhoz im Bezirk Odintsovo im Gebiet Moskau durchgeführt. Die Untersuchungsergebnisse sind in Tabelle 1 dargestellt.

Tabelle 1

| № | № des Kalbes | gemessener Wert | Befund |
|---|---|---|---|
| 1 | 1260 | 1760 | gesund |
| 2 | 1265 | 1795 | gesund |
| 3 | 1263 | 1680 | gesund |
| 4 | 1264 | 2100 | r. Verdacht |
| 5 | 1262 | 1480 | Verdacht |
| 6 | 1252 | 2120 | krank-Verdacht |
| 7 | 1259 | 3400 | krank |
| 8 | 1268 | 1720 | gesund |
| 9 | 1258 | 4500 | krank |
| 10 | 1266 | 6200 | krank |
| 11 | 1267 | 6150 | krank |

Die Untersuchungsergebnisse der Kälber fielen mit den früher vorliegenden Blut- und Urintestergebnissen zusammen.

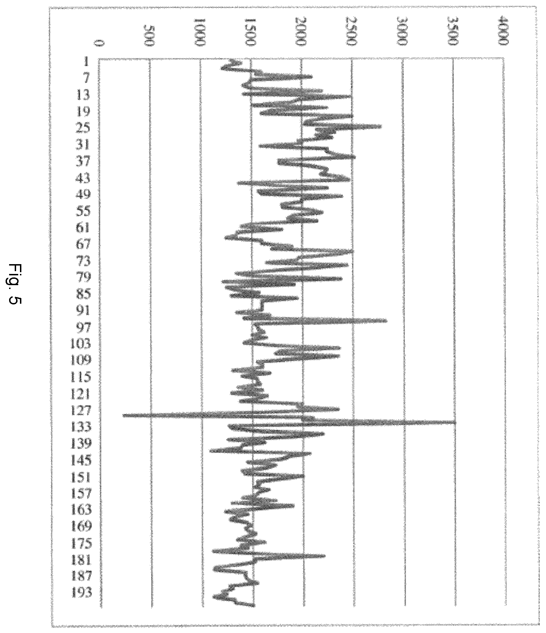

Fig. 5 zeigt ein Messdiagramm für die erste Kuhherde. Auf der X-Achse befindet sich die Seriennummer einer Kuh während der Untersuchung. Auf der Y-Achse ist der gemessene Leitfähigkeitswert in nSm (Nano Siemens). Die Untersuchungsergebnisse der ersten Kuhherde ermöglichten es, kranke Kühe (10 %) und eine Risikogruppe (15 %) zu identifizieren.

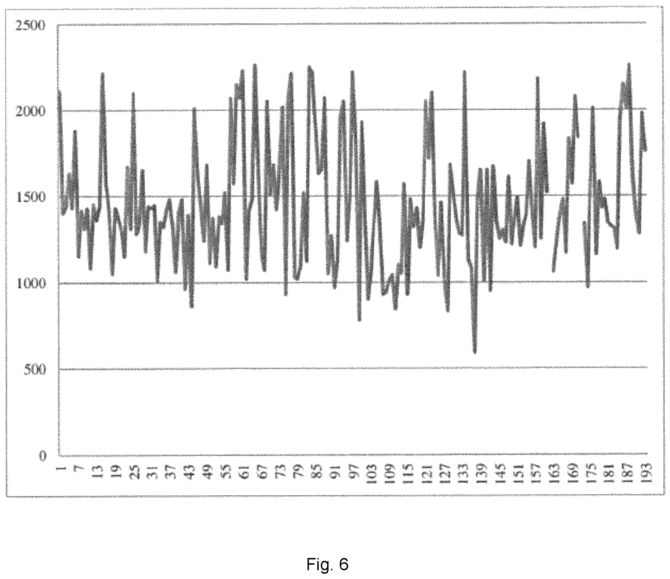

Fig. 6 zeigt ein Messdiagramm für die zweite Kuhherde. Die Untersuchungsergebnisse der zweiten Kuhherde haben Kranke (20 %) und eine erhebliche Risikogruppe (25 %) gezeigt und die Wirksamkeit einer medikamentösen Behandlung in Bezug auf die erste Kuhherde bestätigt.

Das vom vorgeschlagenen Sender erzeugte elektromagnetische Feld ist in einer Ebene symmetrisch. Dazu sind Platten und Löcher runder Form verwendet. In diesem Zusammenhang wird der subjektive Faktor ausgeschlossen, der mit der Orientierung des Senders verbunden ist. Das elektromagnetische Feld ist auch zwischen den Platten konzentriert und ist durch eine große Platte begrenzt, innerhalb deren die zweite Platte angeordnet ist. Daher fallen das Ausstrahlungsfeld und das Absorptionsfeld praktisch zusammen. Die Ausstrahlungsleistung bei diesem Verfahren überschreitet 1 Mikrowatt nicht und ist unter Berücksichtigung der Frequenz von nicht mehr als 20 kHz ein sicheres Untersuchungsverfahren. Diese Leistung ist um Größenordnungen geringer als die Ausstrahlungsleistung der Kopfhörer moderner Geräte im gleichen Frequenzbereich.

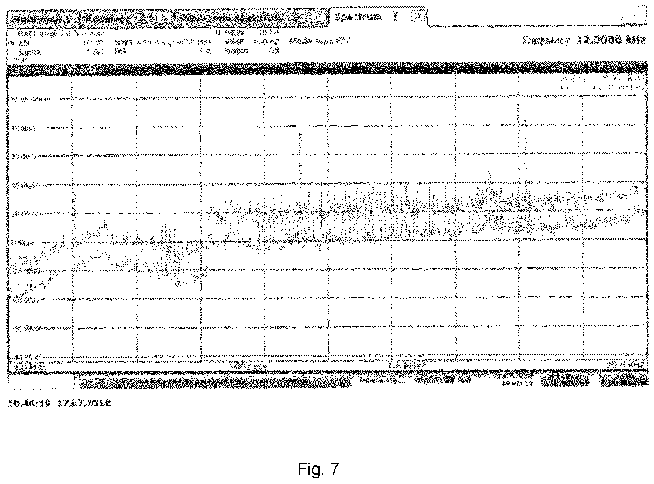

Die Spezialisten der Gesellschaft "RODE & SCHWARTZ RUS" haben das Spektrum und die Ausstrahlungsleistung der CD-Vorrichtung mit einem R&S FSV (Roshde & Schwarz) Spektrum- und Signalanalysator und einer aktiven Stabantenne zur Messung der Stärke des elektrischen Feldes R&S HFH2-Z6E (Roshde & Schwarz) bei einem Abstand von 1 cm vom Sender der CD-Vorrichtung gemessen. Fig. 7 zeigt das entsprechende Spektrumdiagramm. Das Spektrum zeigt Spitzen bei Frequenzen von ~5,65 kHz ~18 dB$\mu$V. Addieren wir zum Pegel (Niveau) mindestens +10 dB ~28 dB$\mu$V, da der Antennenkalibrierkoeffizient 10 dB vom Analysator mit 8,3 kHz berücksichtigt wird, erhalten wir weiter ~11,3 kHz ~38 dB$\mu$V, dann ~17 kHz ~42 dB$\mu$V (Schrittteilung nach der Frequenz 1,6 kHz).

Somit stellt die Vorrichtung eine sichere Diagnose sicher und lässt den Einfluss subjektiver Faktoren bei der Messung ausschließen, wodurch die Möglichkeit einer objektiven Diagnose mit der Feststellung der Abweichungen von der Norm oder festgelegten Eigenschaften sichergestellt wird. Die Vorrichtung kann angepasst werden, um lokale Pathologien biologischer Gewebe, innerer Organe bei den Bauchoperationen zu bestimmen, um die Pathologie von Hautbereichen zu bestimmen, welche die Abweichungen infolge der Störungen von Flüssigkeitsbilanz, Blutfluss und Lymphfluss aufweisen. Dies schließt die Möglichkeit einer mechanischen und thermischen Schädigung des untersuchbaren Organs bei den Messungen aus.

**Patentansprüche**

1. Sender (1) aus Flachelektroden, der die Erzeugung eines torodialen elektromagnetischen Feldes ermöglicht, welches in einer Ebene symmetrisch ist, wobei der Sender aus mehreren Platten besteht, die in derselben Ebene angeordnet sind, wobei mindestens eine der Platten in ihrer Ebene Löcher hat, in denen andere Platten angeordnet sind, und die anderen Platten alle elektrisch miteinander verbunden und von der Platte mit Löchern isoliert sind, und wobei die Platten als Elektroden ausgebildet sind.

**Claims**

1. Transmitter (1) made of flat electrodes, which enables the generation of a toroidal electromagnetic field that is symmetrical in one plane, wherein the transmitter consists of multiple plates arranged in the same plane, wherein at least one of the plates has holes in its plane in which other plates are arranged, and the other plates are all electrically connected to each other and insulated from the plate with holes, and wherein the plates are designed as electrodes.

**Revendications**

1. Émetteur (1) composé d'électrodes plates, permettant la génération d'un champ électromagnétique toroïdal symétrique dans un plan, l'émetteur étant constitué de plusieurs plaques disposées dans le même plan, où au moins une des plaques comporte des trous dans son plan dans lesquels d'autres plaques sont disposées, et les autres plaques sont toutes électriquement connectées entre elles et isolées de la plaque avec des trous, et où les plaques sont conçues comme des électrodes.

Fig. 1

(Stand der Technik)

Fig. 2

Fig. 3

Fig. 4

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2112416 A
- US 2002087201 A1
- DE 3884136 T2
- US 2007179584 A1
- RU 2287891
- RU 2617270
- RU 2366360

**In der Beschreibung aufgeführte Nicht-Patentliteratur**

- **TIMOFEEV GA**. Methoden der apparativen Untersuchung der menschlichen Haut. *Kosmetik und Medizin*, 2005, vol. 4, 28-36
- **KOROLKOVA T. N.** ; **MATYTSIN V. O.** ; **TURKOVSKY I. I.** ; **KHARIN V. N.** Perspektiven der Untersuchung des Wasseraustausches der menschlichen Haut mit der Methode der EHF-Dielektrometrie. *Experimentelle und klinische Dermatokosmetologie*, 2003, vol. 1 (1), 17-192
- **HERAUSGEGEBEN VON G.E. TRUFANOV - M.** Strahlendiagnostik: Lehrbuch T. 1.. *GEOTAR-Media*, 2009
- **EMRAN SHEKI et al.** Concentric Ring Probe for Bioimpedance Spectroscopic Measurements: Design and ex vivio Feasability Testing on Pork Oral Tissues. *SENSORS*, 10 October 2018, vol. 18 (10), 3378
- Theorie des elektromagnetischen Feldes und die Grundlagen der Funkwellenausbreitung. **LAVROV VM**. Lehrbuch für Universitäten für Kommunikation. Verlag, 1964, 231-233
- **BESSONOV L.A.** Theoretische Grundlagen der Elektrotechnik: Elektromagnetisches Feld. Lehrbuch für Universitätsstudenten, 1978, vol. 7, 174-175
- **TAMM I.E.** Grundlagen der Elektrizitätslehre. Lehrbuch für Universitäten, 2003, vol. 11, 468-470
- **LI KALAKUTSKIY** ; **SA AKULOV** ; **AA FEDOTOV.** Elektronische Ressource]: Elektron, Lehrbuch, Handbuch
- Ministerium für Bildung und Wissenschaft Russlands, Samar. Staats- Luft- und Raumfahrt un-t sie. **S. P. KOROLEVA**. Elektron, Text und Grafik. nationale Forschungsuniversität, 2011, 9
- **SIEHE BESSONOV L.A.** Theoretische Grundlagen der Elektrotechnik: Elektromagnetisches Feld. Lehrbuch für Universitätsstudenten, 1978, vol. 7, 138-139
- **SIEHE BESSONOV L.A.** Theoretische Grundlagen der Elektrotechnik: Elektromagnetisches Feld. Lehrbuch für Universitätsstudenten, 1978, vol. 7, 175
- **SIEHE BESSONOV L.A.** Theoretische Grundlagen der Elektrotechnik: Elektromagnetisches Feld. Lehrbuch für Universitätsstudenten, 1978, vol. 7, 128-139
- **SIEHE BESSONOV LA**. Theoretische Grundlagen der Elektrotechnik: Elektromagnetisches Feld. Lehrbuch für Universitätsstudenten, 1978, vol. 7, 150
- **LI KALAKUTSKIY**. Grundlagen der Impuls-Impedanzmessung biologischer Gewebe. FBGOU VPO Samara State University, 67
- **B.I. SEDUNOV** ; **D. A. FRANK-KAMENETSKY**. Dielektrizitätskonstante biologischer Objekte. *Fortschritte in den physikalischen Wissenschaften*, April 1963, vol. 4